# EUROPEAN PATENT APPLICATION

(11) **EP 4 789 602 A1**
(43) Date of publication of application: **12.08.2026**
(21) Application number: 25758195.9
(22) Date of filing: 30.01.2025
(51) Int. Cl.: A61B 3/06, A61B 5/378

(54) **COLOR EVALUATION SYSTEM, COLOR EVALUATION METHOD, PRODUCTION METHOD FOR DYE, AND COLOR EVALUATION PROGRAM**

(30) Priority: 22.02.2024 WO PCT/JP2024/006625; 26.06.2024 JP 2024102766
(71) Applicant: Resonac Corporation, Tokyo 105-7325 (JP)
(72) Inventor: SAKAGUCHI, Suguru, Tokyo 105-7325 (JP); TOMITA, Miyuki, Tokyo 105-7325 (JP)
(74) Representative: Strehl & Partner mbB
(86) International application number: PCT/JP2025/003088
(87) International publication number: WO 2025/177797

(57) **Abstract**

A color evaluation system includes: an acquisition unit configured to acquire reference color information relating to a reference color, a subject model indicating recognition of colors by a subject, and a reference model indicating recognition of colors by a reference observer; a calculation unit configured to calculate a subject response indicating recognition of the reference color by the subject based on the reference color information and the subject model, and to calculate a reference response indicating recognition of the reference color by the reference observer based on the reference color information and the reference model; and a comparison unit configured to calculate a difference between the subject response and the reference response as a response difference.

## Description

### Technical Field

An aspect of the present disclosure relates to a color evaluation system, a color evaluation method, a method for producing a colorant, and a color evaluation program.

### Background Art

Information processing related to color vision has been known in the related art. For example, a color processing device described in Patent Literature 1 acquires a color value to be subjected to color processing, calculates a color vision degree coefficient representing a degree of color vision deficiency based on a distribution of the number of persons in examination results obtained by a color vision examination conducted on a predetermined group, calculates a color conversion coefficient used for converting the acquired color value based on a correspondence between the color vision degree coefficient and sensitivity characteristics of L cones, M cones, and S cones, and performs color conversion processing on the color value using the color conversion coefficient.

### Citation List

### Patent Literature

[Patent Literature 1] Japanese Patent No. 5924289

### Summary of Invention

### Technical Problem

There is a demand for a mechanism for quantitatively evaluating colors from the perspective of human color vision.

### Solution to Problem

A color evaluation system according to an aspect of the present disclosure includes: an acquisition unit configured to acquire reference color information relating to a reference color, a subject model indicating recognition of colors by a subject, and a reference model indicating recognition of colors by a reference observer; a calculation unit configured to calculate a subject response indicating recognition of the reference color by the subject based on the reference color information and the subject model, and to calculate a reference response indicating recognition of the reference color by the reference observer based on the reference color information and the reference model; and a comparison unit configured to calculate a difference between the subject response and the reference response as a response difference.

A color evaluation method according to an aspect of the present disclosure is executed by a color evaluation system including at least one processor. The color evaluation method includes: acquiring reference color information relating to a reference color, a subject model indicating recognition of colors by a subject, and a reference model indicating recognition of colors by a reference observer; calculating a subject response indicating recognition of the reference color by the subject based on the reference color information and the subject model, and calculating a reference response indicating recognition of the reference color by the reference observer based on the reference color information and the reference model; and calculating a difference between the subject response and the reference response as a response difference.

A color evaluation program according to an aspect of the present disclosure causes a computer to execute: acquiring reference color information relating to a reference color, a subject model indicating recognition of colors by a subject, and a reference model indicating recognition of colors by a reference observer; calculating a subject response indicating recognition of the reference color by the subject based on the reference color information and the subject model, and calculating a reference response indicating recognition of the reference color by the reference observer based on the reference color information and the reference model; and calculating a difference between the subject response and the reference response as a response difference.

In such aspects, the recognition of the reference color by the subject is calculated as the subject response, the recognition of the reference color by the reference observer is calculated as the reference response, and the difference between these two responses is calculated as the response difference. The response difference quantitatively indicates the discrepancy in recognition of the reference color between the subject and the reference observer. Therefore, by using the response difference, a color (reference color) can be quantitatively evaluated from the perspective of color vision.

### Advantageous Effects of Invention

According to an aspect of the present disclosure, a color can be quantitatively evaluated from the perspective of human color vision.

### Brief Description of Drawings

FIG. 1 is a diagram showing an example of a functional configuration of a color evaluation system.
FIG. 2 is a flowchart showing an example of an operation of the color evaluation system.
FIG. 3 is a graph showing an example of sensitivity characteristics of L cones, M cones, and S cones.
FIG. 4 is a flowchart showing an example of an operation of the color evaluation system.

### Description of Embodiments

Hereinafter, various examples in the present disclosure will be described in detail with reference to the accompanying drawings. In the description of the drawings, the same or equivalent elements are denoted by the same reference numerals, and a redundant description thereof will be omitted.

### [Outline of System]

A color evaluation system according to the present disclosure is a computer system for quantitatively evaluating colors from the perspective of human color vision. The color evaluation system may also be said to be a computer system that evaluates colors in consideration of color vision diversity. Color vision diversity refers to a situation in which recognition or discrimination of colors differs among people. The color evaluation system may be used for the purpose of facilitating common recognition of colors among people having different types of color vision or promoting mutual understanding regarding recognition or discrimination of colors.

As types of color vision, there are five types: C-type (Common), P-type (Protanope), D-type (Deuteranope), T-type (Tritanope), and A-type (Achromatic).

The C-type is a group having three types of cones: L cones, M cones, and S cones. The L cones are photoreceptor cells that respond with high sensitivity to light near long wavelengths such as red. The M cones are photoreceptor cells that respond with high sensitivity to light near medium wavelengths such as green. The S cones are photoreceptor cells that respond with high sensitivity to light near short wavelengths such as blue. In the case of Japanese people, 95% of males and 99% of females belong to the C-type. People of the C-type are also referred to as persons with normal color vision.

On the other hand, people of the P-type, D-type, T-type, and A-type are also referred to as persons with color vision deficiency. The P-type is a group consisting of protanopia in which L cones are absent and protanomaly in which the sensitivity of L cones is shifted to resemble the sensitivity of M cones. The D-type is a group consisting of deuteranopia in which M cones are absent and deuteranomaly in which the sensitivity of M cones is shifted to resemble the sensitivity of L cones. The T-type is a group in which S cones are absent. The A-type is a group having only one type of cone or having no cones at all. Among persons with color vision deficiency, the P-type and D-type account for the majority, and the proportions of the T-type and A-type are extremely small.

As an example of promoting the above-described common recognition or mutual understanding, the color evaluation system may be used to evaluate a difference in recognition between a person with color vision deficiency and a person with normal color vision with respect to a certain single color. Alternatively, the color evaluation system may be used to evaluate whether a certain color causes a discrepancy in recognition between a person with color vision deficiency and a person with normal color vision. Alternatively, the color evaluation system may be used to adjust a reference spectrum, which is a spectrum of the reference color, to provide a color that is recognized in the same manner by both a person with normal color vision and a person with color vision deficiency. The reference spectrum is an example of reference color information relating to the reference color. The color evaluation system may be used for the purpose of promoting common recognition or mutual understanding of colors among people of the C-type, P-type, D-type, T-type, and A-type.

### [Configuration of System]

The color evaluation system is constituted by one or more computers. In a case where a plurality of computers are used, these computers are connected via a communication network such as the Internet or an intranet, to logically construct one color evaluation system.

A computer constituting the color evaluation system generally includes, as hardware devices, a processor, a memory, a communication interface, an input device, and an output device. Examples of the processor include a CPU and a GPU. The memory may be constituted by a flash memory, a hard disk, and the like. The communication interface may be constituted by a network card or a wireless communication module. Examples of the input device include a keyboard, a pointing device, a touch panel, a microphone, a sensor, and a camera. Examples of the output device include a monitor, a touch panel, a head-mounted display (HMD), and a speaker.

A color evaluation program for causing a computer to function as the color evaluation system includes program code for implementing each functional module of the color evaluation system. The color evaluation program may be recorded on a tangible recording medium such as a CD-ROM, a DVD-ROM, or a semiconductor memory in a non-transitory manner and then provided. Alternatively, the color evaluation program may be provided as data signals superimposed on carrier waves via a communication network. The provided color evaluation program is stored in, for example, the memory.

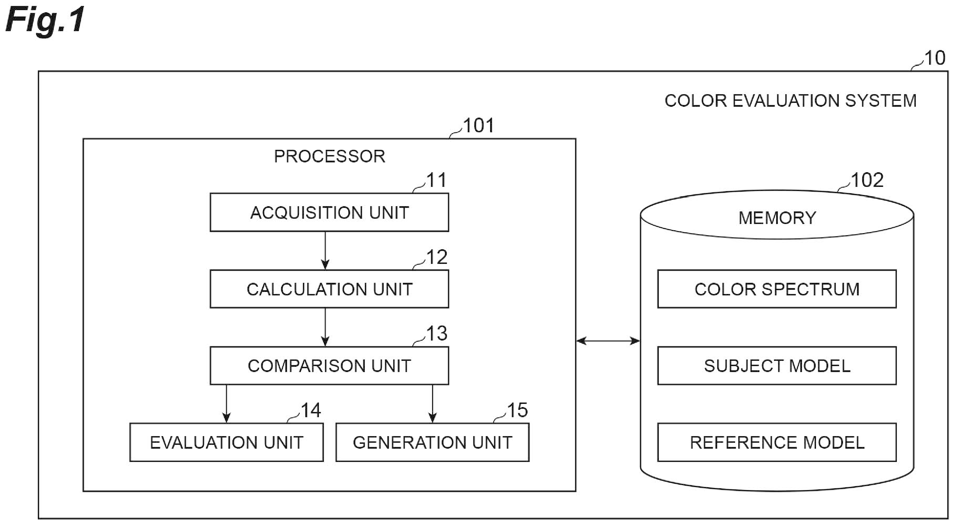

A configuration of a color evaluation system 10 according to one example will be described with reference to FIG. 1. FIG. 1 is a diagram showing a functional configuration of the color evaluation system 10.

The color evaluation system 10 includes a processor 101 and a memory 102. In one example, the processor 101 functions as an acquisition unit 11, a calculation unit 12, a comparison unit 13, an evaluation unit 14, and a generation unit 15. The memory 102 stores in advance a color spectrum which is spectral data of a color, a subject model indicating recognition of colors by a subject, and a reference model indicating recognition of colors by a reference observer. The memory 102 may store one or more color spectra, one or more subject models, and one or more reference models. Both the subject model and the reference model are color vision models that represent a color vision system of a person by an algorithm or data. The color vision system is constituted by including the eyeball, the optic nerve, the visual cortex, and the like.

The subject refers to a person assumed for the purpose of evaluating colors, and the reference observer refers to a person assumed for the purpose of comparison with the subject. The subject may be a person with normal color vision or may be a person with color vision deficiency of a specific type such as the P-type or the D-type. For example, in a case where a specific person with color vision deficiency is selected as the subject, a person with normal color vision is assumed as the reference observer, and in a case where a person with normal color vision is selected as the subject, a person with color vision deficiency of a specific type is assumed as the reference observer. Reference models may be prepared for a person with normal color vision and a person with color vision deficiency, respectively. In a case where a person with color vision deficiency is assumed as the reference observer, the reference model may be prepared for each type of color vision, and for example, a reference model for the P-type, a reference model for the D-type, a reference model for the T-type, and a reference model for the A-type may be prepared. Furthermore, reference models in which a difference in degree such as severe or mild color vision deficiency is considered may be prepared.

The acquisition unit 11 is a functional module that acquires a reference spectrum, a subject model, and a reference model. The calculation unit 12 is a functional module that calculates a subject response indicating recognition of the reference color by the subject based on the reference spectrum and the subject model, and calculates a reference response indicating recognition of the reference color by the reference observer based on the reference spectrum and the reference model. The comparison unit 13 is a functional module that calculates a difference between the subject response and the reference response as a response difference. The evaluation unit 14 is a functional module that evaluates the reference color based on the response difference. The generation unit 15 is a functional module that generates an adjusted spectrum, which is a spectrum of a color having a response difference smaller than that of the reference spectrum, based on the reference spectrum and the response difference. A color having the adjusted spectrum is a color that is recognized in the same manner by both the subject and the reference observer, that is, a color that is recognized in the same manner by both a person with normal color vision and a person with color vision deficiency.

### [Operation of System]

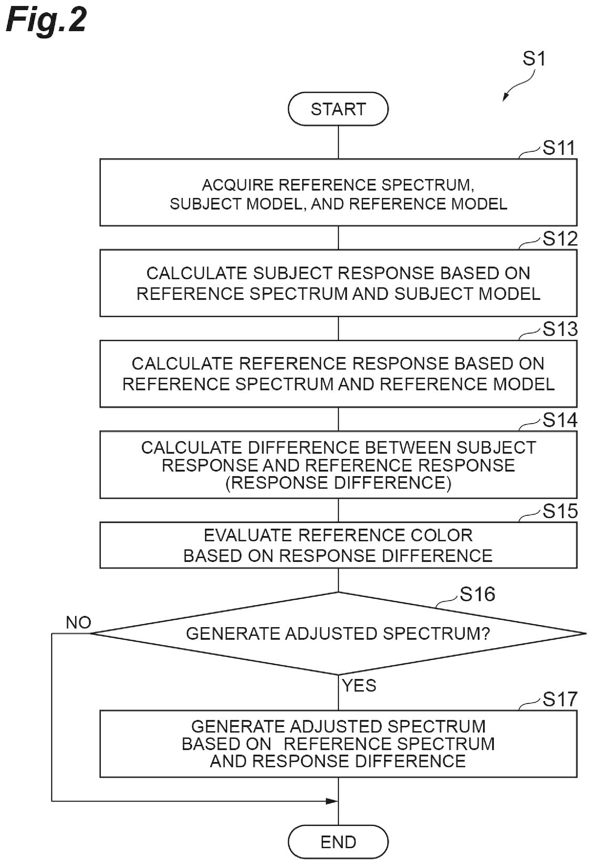

A color evaluation method executed by the color evaluation system 10 will be described with reference to FIG. 2. FIG. 2 is a flowchart showing an example of an operation of the color evaluation system 10 as a processing flow S1.

In step S11, the acquisition unit 11 acquires a reference spectrum, a subject model, and a reference model. In one example, a user of the color evaluation system 10 inputs a reference color, a type of color vision of the subject, and a type of color vision of the reference observer. The acquisition unit 11 receives the input and reads, from the memory 102, a reference spectrum which is a spectrum of the reference color, a subject model corresponding to the type of color vision of the subject, and a reference model corresponding to the type of color vision of the reference observer. Alternatively, the acquisition unit 11 may directly receive input of the reference spectrum, the subject model, and the reference model. Alternatively, the acquisition unit 11 may receive the reference spectrum, the subject model, and the reference model from another computer.

In one example, the reference spectrum is represented by luminance (cd/m²) at each wavelength (nm) in the visible light region. The reference spectrum may be expressed by a spectrum function indicating a relationship between wavelength and luminance.

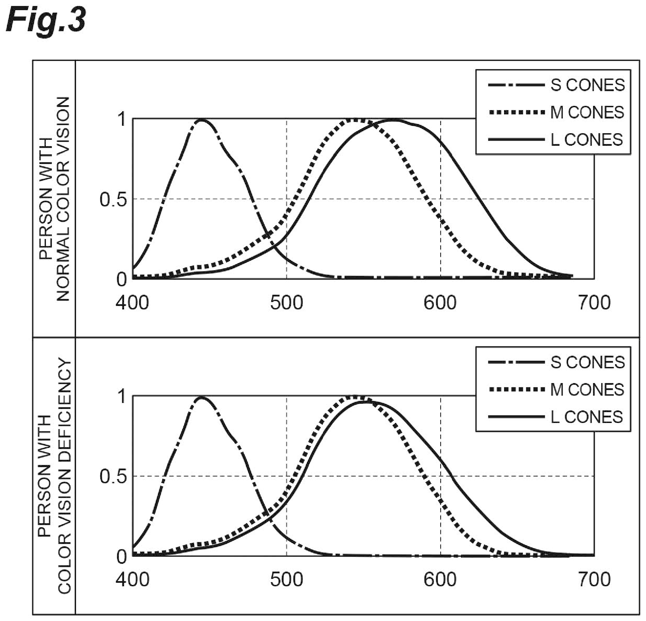

In one example, both the subject model and the reference model indicate sensitivity characteristics of each of L cones, M cones, and S cones. The sensitivity characteristics are also referred to as spectral characteristics. FIG. 3 is a graph showing an example of sensitivity characteristics of each cone for a person with normal color vision and a person with color vision deficiency. The horizontal axis and the vertical axis of the graph indicate wavelength (nm) and relative sensitivity, respectively. The person with color vision deficiency shown in FIG. 3 corresponds to protanomaly. The person with color vision deficiency has a greater degree of overlap between the sensitivity characteristics of M cones and L cones compared to a person with normal color vision (C-type). Such a difference in sensitivity characteristics causes a difference in recognition or discrimination of colors. A color vision model such as that shown in FIG. 3 may be expressed by a sensitivity function indicating a relationship between wavelength and relative sensitivity. The sensitivity function is prepared for each of L cones, M cones, and S cones. In the present disclosure, such a sensitivity function is also referred to as a "cone sensitivity function." The subject model includes a sensitivity function indicating sensitivity characteristics of each of L cones, M cones, and S cones of the subject. The reference model includes a sensitivity function indicating sensitivity characteristics of each of L cones, M cones, and S cones of the reference observer. These color vision models may be generated based on measured values obtained by measuring the sensitivity of L cones, M cones, and S cones of an individual, or may be generated based on representative values for the C-type, protanomaly, deuteranomaly, and the like.

Returning to FIG. 2, in step S12, the calculation unit 12 calculates a subject response indicating recognition of the reference color by the subject based on the reference spectrum and the subject model.

In one example, the calculation unit 12 calculates a subject response indicating responses from each of L cones, M cones, and S cones of the subject. In this example, the subject response defined by a response α from L cones, a response β from M cones, and a response γ from S cones is expressed as (α, β, γ). The calculation unit 12 calculates, for each of L cones, M cones, and S cones of the subject, a response from the cone that has sensed the reference color as follows. The calculation unit 12 calculates, at each wavelength within the visible light region, the product of the relative sensitivity of the cone and the luminance indicated by the reference spectrum. The product may be said to indicate a degree of excitation. The calculation unit 12 obtains a sum of the products at the respective wavelengths as the response from the cone.

Let the wavelength be represented by x, let the sensitivity functions of L cones, M cones, and S cones be represented by f_{L}(x), f_{M}(x), and f_{S}(x), respectively, and let the spectrum function of the reference color be represented by g(x). In this case, the responses α, β, and γ may be expressed by Equations (1) to (3), respectively.$α = \int {f}_{L} \left(x\right) g \left(x\right) dx$ $β = \int {f}_{M} \left(x\right) g \left(x\right) dx$ $γ = \int {f}_{S} \left(x\right) g \left(x\right) dx$

That is, the calculation unit 12 calculates, as the subject response (α, β, γ), the integral of the product of the sensitivity function and the spectrum function for each cone of the subject.

In step S13, the calculation unit 12 calculates a reference response indicating recognition of the reference color by the reference observer based on the reference spectrum and the reference model. In one example, the calculation unit 12 calculates a reference response indicating responses from each of L cones, M cones, and S cones of the reference observer. In this example, the reference response defined by a response α' from L cones, a response β' from M cones, and a response γ' from S cones is expressed as (α', β', γ'). In the same manner as in the case of calculating the subject response, the calculation unit 12 calculates, for each of L cones, M cones, and S cones of the reference observer, a response from the cone that has sensed the reference color using the above Equations (1) to (3), to obtain the reference response (α', β', γ'). That is, the calculation unit 12 calculates, as the reference response (α', β', γ'), the integral of the product of the sensitivity function and the spectrum function for each cone of the reference observer.

In step S14, the comparison unit 13 calculates a difference between the subject response and the reference response as a response difference. In a case where the subject response and the reference response indicate responses from each of L cones, M cones, and S cones, the comparison unit 13 calculates a difference between the subject response and the reference response for each of L cones, M cones, and S cones, and calculates the response difference based on a combination of the difference for L cones, the difference for M cones, and the difference for S cones. For example, the comparison unit 13 obtains a response difference (α-α', β-β', γ-γ'). Alternatively, the comparison unit 13 may calculate the response difference as a ratio based on the reference response, in which case the response difference may be expressed as $\left\{\left(α-α′\right)/α′, \left(β-β′\right)/β′, \left(γ-γ′\right)/γ′\right\} .$

In step S15, the evaluation unit 14 evaluates the reference color based on the response difference. For example, in a case where the response difference is equal to or greater than a predetermined threshold value Th, the evaluation unit 14 determines that the reference color is a color that is recognized differently by the subject and the reference observer. On the other hand, in a case where the response difference is less than the threshold value Th, the evaluation unit 14 determines that the reference color is a color that is recognized in the same manner by the subject and the reference observer. The threshold value Th is set so as to determine a color that is recognized in the same manner by the subject and the reference observer. The evaluation unit 14 outputs the determination result. The evaluation unit 14 may display the determination result on a display device, may store the determination result in a predetermined storage device such as the memory 102, or may transmit the determination result to another computer system.

In a case where cone responses are used, the evaluation unit 14 may use, as the threshold value Th, a combination of a threshold value Th_{L} for L cones, a threshold value Th_{M} for M cones, and a threshold value Th_{S} for S cones. In this case, the evaluation unit 14 compares, for each of L cones, M cones, and S cones, the difference for the cone with the threshold value for the cone. In a case where the difference is equal to or greater than the threshold value for at least one of the three types of cones, the evaluation unit 14 determines that the reference color is a color that is recognized differently by the subject and the reference observer. On the other hand, in a case where the difference is less than the threshold value for all three types of cones, the evaluation unit 14 determines that the reference color is a color that is recognized in the same manner by the subject and the reference observer.

In step S16, the generation unit 15 determines whether to generate an adjusted spectrum. In one example, in a case where the evaluation unit 14 has determined that the reference color is a color that is recognized differently by the subject and the reference observer, the generation unit 15 determines to generate an adjusted spectrum (YES in step S16). In this case, the process proceeds to step S17. On the other hand, in a case where the evaluation unit 14 has determined that the reference color is a color that is recognized in the same manner by the subject and the reference observer, the generation unit 15 determines not to generate an adjusted spectrum (NO in step S16). In this case, the processing flow S1 ends without step S17 being executed.

In step S17, the generation unit 15 generates an adjusted spectrum, which is a spectrum of a color having a response difference smaller than that of the reference spectrum, based on the reference spectrum and the response difference. In one example, the generation unit 15 changes at least a part of the reference spectrum to generate a candidate spectrum. Then, the generation unit 15 calculates the above Equations (1) to (3) based on the spectrum function of the candidate spectrum and the sensitivity functions of L cones, M cones, and S cones of the subject, to calculate a subject response (α, β, γ) corresponding to the candidate spectrum. Also, the generation unit 15 calculates the above Equations (1) to (3) based on the spectrum function of the candidate spectrum and the sensitivity functions of L cones, M cones, and S cones of the reference observer, to calculate a reference response (α', β', γ') corresponding to the candidate spectrum. Subsequently, the generation unit 15 calculates a response difference between the subject response (α, β, γ) and the reference response (α', β', γ'). The generation unit 15 repeats the calculation of the subject response (α, β, γ) and the reference response (α', β', γ') and the calculation of the response difference while changing at least a part of the reference spectrum. Then, the generation unit 15 selects, as the adjusted spectrum, a candidate spectrum for which a response difference smaller than the response difference for the reference spectrum has been obtained. For example, the generation unit 15 selects, as the adjusted spectrum, a candidate spectrum for which the response difference is equal to or less than the above threshold value Th. The adjusted spectrum selected based on the threshold value Th may be said to be a color spectrum in which the subject response matches or approximates the reference response.

The generation unit 15 outputs the adjusted spectrum. The generation unit 15 may display the adjusted spectrum on a display device, may store the adjusted spectrum in a predetermined storage device such as the memory 102, or may transmit the adjusted spectrum to another computer system. Alternatively, the generation unit 15 may output the adjusted spectrum to a producing apparatus, which is an apparatus or a group of apparatuses that produces a colorant. A colorant refers to a substance that imparts color to an object. The colorant may be a dye or a pigment. The producing apparatus may be a component of the color evaluation system 10 or may be provided outside the color evaluation system 10. The producing apparatus refers to the adjusted spectrum, selects one or more colorant materials from a plurality of colorant materials prepared in advance, and determines a mixing ratio for each of the selected colorant materials. Then, the producing apparatus mixes the selected one or more colorant materials at the determined mixing ratio to produce a colorant. The producing apparatus may generate a colorant according to one or more colorant materials and a mixing ratio selected by an operator. In any case, the producing apparatus produces a colorant based on the adjusted spectrum. The colorant has the adjusted spectrum and can contribute to color expression in consideration of color vision diversity.

### [Modification Examples]

The technology according to the present disclosure has been described in detail above based on various examples. However, the present disclosure is not limited to the above examples. The technology according to the present disclosure may be modified in various ways without departing from the gist of the present disclosure.

### (First Modification Example)

In the above examples, the subject model and the reference model include cone sensitivity functions. As another example, both the subject model and the reference model may include a sensitivity function indicating a relationship between a color spectrum and a brain wave. In the present disclosure, the sensitivity function is also referred to as a "brain wave sensitivity function." The brain wave sensitivity function may be a sensitivity function indicating a relationship between a color spectrum and a steady state visual evoked potential (Steady State Visual Evoked Potential (SSVEP)) obtained by processing a brain wave by frequency analysis such as Fourier transform. The SSVEP is represented as a power spectral density obtained by Fourier-transforming with frequency components of a brain wave signal of an observer when the observer is viewing a color that changes periodically. The subject model is generated based on a brain wave of the subject, and the reference model is generated based on a brain wave of the reference observer.

In a case where the brain wave sensitivity function is used, the calculation unit 12 inputs the reference spectrum to the sensitivity function of the subject to calculate a subject response based on the brain wave of the subject. Also, the calculation unit 12 inputs the reference spectrum to the sensitivity function of the reference observer to calculate a reference response based on the brain wave of the reference observer. The comparison unit 13 calculates a difference between these two responses as a response difference. Even in a case where the brain wave sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

### (Second Modification Example)

The brain wave sensitivity function may be a sensitivity function f_{B} indicating a ratio of a magnitude of the SSVEP (second SSVEP) of a target person when the target person has visually recognized a change from the reference color to another predetermined color, to a magnitude of the SSVEP (first SSVEP) of the target person when the target person has visually recognized a change from a predetermined first gray to a predetermined second gray. That is, f_{B} = (magnitude of second SSVEP) / (magnitude of first SSVEP). The first gray and the second gray differ in shade. The other color may be set in advance in accordance with the reference color, and may be, for example, a color located in a symmetrical relationship with the reference color in a color coordinate system having a cone response difference as an axis. The coordinate system is also referred to as an MB-DKL space. Alternatively, the other color may be a color that is difficult for the target person to distinguish from the reference color, or may be a color located on the same confusion line as the reference color. The ratio obtained as a response by the sensitivity function f_{B} is a value greater than 0. The SSVEP corresponds to a frequency component of a brain wave of a subject who has visually recognized a periodic change in color. The brain wave is measured at one or more locations, for example, at four or more locations, corresponding to the position of the visual cortex in the occipital region. If the target person cannot perceive a difference between the reference color and the other color, the brain wave component becomes small, and the response obtained from the sensitivity function f_{B} becomes a value close to 0. On the other hand, if the target person can perceive a difference between the reference color and the other color, the brain wave component becomes large, and the response obtained from the sensitivity function f_{B} becomes a large value.

The subject model includes the sensitivity function f_{B} for the subject, and the reference model includes the sensitivity function f_{B} for the reference observer. In a case where these sensitivity functions are used, the acquisition unit acquires a first SSVEP and a second SSVEP of the subject who has visually recognized the reference color, and a first SSVEP and a second SSVEP of the reference observer who has visually recognized the reference color. Also, the acquisition unit acquires the subject model and the reference model. The calculation unit calculates, as the subject response, a ratio obtained by substituting the first SSVEP and the second SSVEP of the subject into the sensitivity function f_{B} for the subject. Similarly, the calculation unit calculates, as the reference response, a ratio obtained by substituting the first SSVEP and the second SSVEP of the reference observer into the sensitivity function f_{B} for the reference observer. The comparison unit calculates a difference between the subject response and the reference response as a response difference. Even in a case where the sensitivity function f_{B} is used, the evaluation unit may evaluate the reference color based on the response difference, the generation unit may generate an adjusted spectrum based on the reference spectrum and the response difference, and the producing apparatus may produce a colorant based on the adjusted spectrum.

### (Third Modification Example)

As another example of processing based on brain waves, both the subject model and the reference model may include a database indicating, for a plurality of sample colors, a relationship between a sample color and the SSVEP of a target person who has visually recognized the sample color. Each record of the database includes a sample color and the SSVEP of a target person who has visually recognized the sample color. The database may be prepared for each target person, and therefore may be prepared separately for the subject and the reference observer. Alternatively, the database may be a common database prepared by collecting the correspondence relationship for a plurality of sample colors from a plurality of target persons and statistically processing the correspondence relationship for each sample color. The common database is prepared by calculating, for each of a plurality of sample colors, a statistical value (for example, an average) of the SSVEPs of a plurality of target persons and generating a correspondence relationship between the sample color and the statistical value. The common database is used as both the subject model and the reference model.

In a case where the database is used, the acquisition unit acquires the SSVEP of the subject who has visually recognized the reference color and the SSVEP of the reference observer who has visually recognized the reference color. Also, the acquisition unit acquires the subject model and the reference model. Accessing the database is an example of acquiring the subject model and the reference model. The calculation unit refers to the database corresponding to the subject and identifies, as the subject response, a sample color corresponding to the SSVEP of the subject that has been acquired. The calculation unit may select two or more SSVEPs close to the acquired SSVEP from the database, linearly combine the two or more SSVEPs to generate one SSVEP, and identify a sample color corresponding to the generated SSVEP as the subject response. The calculation unit refers to the database corresponding to the reference observer and identifies, as the reference response, a sample color corresponding to the SSVEP of the reference observer that has been acquired. In the same manner as in the case of the subject response, the calculation unit may perform linear combination to identify the reference response. The comparison unit calculates, as the response difference, a distance in a color space (for example, an HSV color space) between the sample color indicated as the subject response and the sample color indicated as the reference response. Even in a case where the database indicating the correspondence relationship between sample colors and SSVEPs is used, the evaluation unit may evaluate the reference color based on the response difference, the generation unit may generate an adjusted spectrum based on the reference spectrum and the response difference, and the producing apparatus may produce a colorant based on the adjusted spectrum.

### (Fourth Modification Example)

Both the subject model and the reference model may include a trained model that receives an input of an SSVEP and estimates a sample color. The trained model is generated by machine learning, which is a technique of autonomously finding laws or rules by iteratively learning based on given information. In this machine learning, training data indicating a correspondence relationship between a sample color and the SSVEP of a target person who has visually recognized the sample color, such as data stored in the above-described database, is used. In this machine learning, the sample color is used as a ground truth. Generation of the trained model may be executed by a learning unit of the color evaluation system. Alternatively, a trained model generated by a computer system different from the color evaluation system may be transferred to the color evaluation system. Since the trained model estimates a sample color from an SSVEP, it may be said to indicate a relationship between a sample color and the steady state visual evoked potential of a subject who has visually recognized the sample color, in the same manner as the above-described database. The trained model may be prepared for each target person, and therefore may be prepared separately for the subject and the reference observer. Alternatively, the trained model may be generated so as to be used in common for the subject and the reference observer.

In a case where the trained model is used, the acquisition unit acquires the SSVEP of the subject who has visually recognized the reference color and the SSVEP of the reference observer who has visually recognized the reference color. Also, the acquisition unit acquires the subject model and the reference model. Accessing the trained model is an example of acquiring the subject model and the reference model. The calculation unit inputs the acquired SSVEP of the subject to the trained model corresponding to the subject and identifies, as the subject response, a sample color output from the trained model. The calculation unit may select two or more SSVEPs close to the acquired SSVEP from the above-described database, linearly combine the two or more SSVEPs to generate one SSVEP, and input the generated SSVEP to the trained model. The calculation unit inputs the acquired SSVEP of the reference observer to the trained model corresponding to the reference observer and identifies, as the reference response, a sample color output from the trained model. In the same manner as in the case of the subject response, the calculation unit may perform linear combination. The comparison unit calculates, as the response difference, a distance in a color space (for example, an HSV color space) between the sample color indicated by the subject response and the sample color indicated by the reference response. Even in a case where the trained model that receives an input of an SSVEP and outputs a sample color is used, the evaluation unit may evaluate the reference color based on the response difference, the generation unit may generate an adjusted spectrum based on the reference spectrum and the response difference, and the producing apparatus may produce a colorant based on the adjusted spectrum.

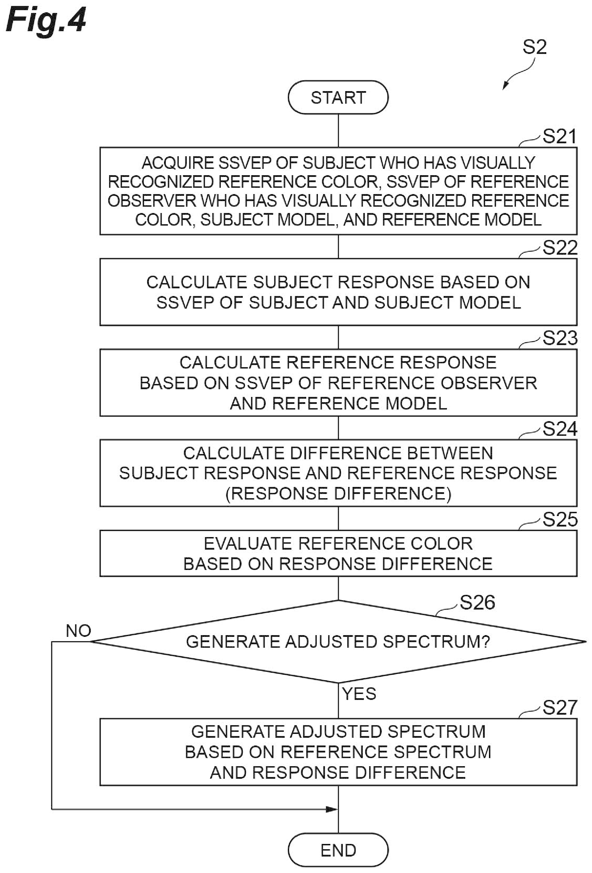

FIG. 4 is a flowchart showing an example of an operation of the color evaluation system corresponding to the second to fourth modification examples as a processing flow S2.

In step S21, the acquisition unit acquires the SSVEP of the subject who has visually recognized the reference color, the SSVEP of the reference observer who has visually recognized the reference color, the subject model, and the reference model. The SSVEP of the subject who has visually recognized the reference color and the SSVEP of the reference observer who has visually recognized the reference color are both examples of reference color information. In step S22, the calculation unit calculates a subject response indicating recognition of the reference color by the subject based on the SSVEP of the subject and the subject model. In step S23, the calculation unit calculates a reference response indicating recognition of the reference color by the reference observer based on the SSVEP of the reference observer and the reference model. The processing of steps S24 to S27 is the same as the processing of steps S14 to S17 in the processing flow S1.

### (Fifth Modification Example)

As another example different from cones and brain waves, it is also conceivable to construct a sensitivity function that reflects a change in pupil size. The pupil size changes depending on how much the attention of the target person is attracted to a certain color. In a case where the target person cannot perceive a difference between a certain color Cx and another color Cd, it is predicted that the pupil size does not change significantly (that is, the amount of change in pupil size is less than a predetermined threshold value). On the other hand, in a case where the target person can perceive a difference between the color Cx and the other color Cd, it is predicted that the pupil size changes (that is, the amount of change in pupil size is equal to or greater than a predetermined threshold value).

The pupil sensitivity function may be a sensitivity function indicating a relationship between a color spectrum and an amount of change in pupil size. In a case where the pupil sensitivity function is used, the calculation unit 12 inputs the reference spectrum to the sensitivity function of the subject to calculate a subject response based on the amount of change in pupil size of the subject. Also, the calculation unit 12 inputs the reference spectrum to the sensitivity function of the reference observer to calculate a reference response based on the amount of change in pupil size of the reference observer. The comparison unit 13 calculates a difference between these two responses as a response difference. Even in a case where the pupil sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

### (Sixth Modification Example)

As yet another example, it is also conceivable to construct a sensitivity function that reflects a change in cerebral blood flow. The cerebral blood flow changes depending on how much the attention of the target person is attracted to a certain color. In a case where the target person cannot perceive a difference between a certain color Cx and another color Cd, it is predicted that the cerebral blood flow does not change significantly (that is, the amount of change in cerebral blood flow is less than a predetermined threshold value). On the other hand, in a case where the target person can perceive a difference between the color Cx and the other color Cd, it is predicted that the cerebral blood flow changes (that is, the amount of change in cerebral blood flow is equal to or greater than a predetermined threshold value).

The cerebral blood flow sensitivity function may be a sensitivity function indicating a relationship between a color spectrum and an amount of change in cerebral blood flow. In a case where the cerebral blood flow sensitivity function is used, the calculation unit 12 inputs the reference spectrum to the sensitivity function of the subject to calculate a subject response based on the amount of change in cerebral blood flow of the subject. Also, the calculation unit 12 inputs the reference spectrum to the sensitivity function of the reference observer to calculate a reference response based on the amount of change in cerebral blood flow of the reference observer. The comparison unit 13 calculates a difference between these two responses as a response difference. Even in a case where the cerebral blood flow sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

### (Seventh Modification Example)

As yet another example, it is also conceivable to construct a sensitivity function that reflects a change in amylase concentration. The amylase concentration changes depending on how much the attention of the target person is attracted to a certain color. In a case where the target person cannot perceive a difference between a certain color Cx and another color Cd, it is predicted that the amylase concentration does not change significantly (that is, the amount of change in amylase concentration is less than a predetermined threshold value). On the other hand, in a case where the target person can perceive a difference between the color Cx and the other color Cd, it is predicted that the amylase concentration changes (that is, the amount of change in amylase concentration is equal to or greater than a predetermined threshold value).

The amylase concentration sensitivity function may be a sensitivity function indicating a relationship between a color spectrum and an amount of change in amylase concentration. In a case where the amylase concentration sensitivity function is used, the calculation unit 12 inputs the reference spectrum to the sensitivity function of the subject to calculate a subject response based on the amount of change in amylase concentration of the subject. Also, the calculation unit 12 inputs the reference spectrum to the sensitivity function of the reference observer to calculate a reference response based on the amount of change in amylase concentration of the reference observer. The comparison unit 13 calculates a difference between these two responses as a response difference. Even in a case where the amylase concentration sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

### (Eighth Modification Example)

As yet another example, it is also conceivable to construct a sensitivity function that reflects a change in gaze. The gaze changes depending on how much the attention of the target person is attracted to a certain color. In a case where the target person cannot perceive a difference between a certain color Cx and another color Cd, it is predicted that the gaze does not change significantly (that is, the amount of change in gaze is less than a predetermined threshold value). On the other hand, in a case where the target person can perceive a difference between the color Cx and the other color Cd, it is predicted that the gaze changes (that is, the amount of change in gaze is equal to or greater than a predetermined threshold value).

The gaze sensitivity function may be a sensitivity function indicating a relationship between a color spectrum and an amount of change in gaze. In a case where the gaze sensitivity function is used, the calculation unit 12 inputs the reference spectrum to the sensitivity function of the subject to calculate a subject response based on the amount of change in gaze of the subject. Also, the calculation unit 12 inputs the reference spectrum to the sensitivity function of the reference observer to calculate a reference response based on the amount of change in gaze of the reference observer. The comparison unit 13 calculates a difference between these two responses as a response difference. Even in a case where the gaze sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

### (Ninth Modification Example)

As yet another example, it is also conceivable to construct a sensitivity function that reflects a change in skin potential. The skin potential changes depending on how much the attention of the target person is attracted to a certain color. In a case where the target person cannot perceive a difference between a certain color Cx and another color Cd, it is predicted that the skin potential does not change significantly (that is, the amount of change in skin potential is less than a predetermined threshold value). On the other hand, in a case where the target person can perceive a difference between the color Cx and the other color Cd, it is predicted that the skin potential changes (that is, the amount of change in skin potential is equal to or greater than a predetermined threshold value).

The skin potential sensitivity function may be a sensitivity function indicating a relationship between a color spectrum and an amount of change in skin potential. In a case where the skin potential sensitivity function is used, the calculation unit 12 inputs the reference spectrum to the sensitivity function of the subject to calculate a subject response based on the amount of change in skin potential of the subject. Also, the calculation unit 12 inputs the reference spectrum to the sensitivity function of the reference observer to calculate a reference response based on the amount of change in skin potential of the reference observer. The comparison unit 13 calculates a difference between these two responses as a response difference. Even in a case where the skin potential sensitivity function is used, in the same manner as in the case where the cone sensitivity function is used, the evaluation unit 14 may evaluate the reference color based on the response difference, the generation unit 15 may generate an adjusted spectrum based on the reference spectrum and the response difference, and the producing apparatus may produce a colorant based on the adjusted spectrum.

The color evaluation system need not execute at least one of the evaluation of the reference color based on the response difference and the generation of the adjusted spectrum. Therefore, the color evaluation system need not include a functional module corresponding to at least one of the evaluation unit 14 and the generation unit 15.

The processing procedure of a method executed by at least one processor is not limited to the above examples. For example, some of the above-described steps may be omitted, or the steps may be executed in a different order. In addition, any two or more of the above-described steps may be combined, or some of the steps may be corrected or deleted. Alternatively, other steps may be executed in addition to each of the above-described steps.

In the comparison between the magnitudes of two numerical values in the present disclosure, either of two criteria of "equal to or greater than" and "greater than" may be used, or either of two criteria of "equal to or less than" and "less than" may be used.

In the present disclosure, an expression "At least one processor executes a first process, executes a second process, ... and executes an n-th process." or an expression corresponding thereto indicates a concept including a case where an entity that executes n processes from the first process to the n-th process, that is, a processor, is changed in the middle. That is, this expression indicates a concept including both a case where all of the n processes are executed by the same processor and a case where the processor is changed according to any policy in the n processes.

### [Appendix]

As will be appreciated from the various examples above, the present disclosure includes the following aspects.

### (Appendix 1)

A color evaluation system comprising:
an acquisition unit configured to acquire reference color information relating to a reference color, a subject model indicating recognition of colors by a subject, and a reference model indicating recognition of colors by a reference observer;
a calculation unit configured to calculate a subject response indicating recognition of the reference color by the subject based on the reference color information and the subject model, and to calculate a reference response indicating recognition of the reference color by the reference observer based on the reference color information and the reference model; and
a comparison unit configured to calculate a difference between the subject response and the reference response as a response difference.

### (Appendix 2)

The color evaluation system according to Appendix 1, further comprising an evaluation unit configured to evaluate the reference color based on the response difference.

### (Appendix 3)

The color evaluation system according to Appendix 1 or 2, further comprising a generation unit configured to generate, based on a reference spectrum which is a spectrum of the reference color and the response difference, an adjusted spectrum, which is a spectrum of a color having a response difference smaller than that of the reference spectrum.

### (Appendix 4)

The color evaluation system according to any one of Appendices 1 to 3,
wherein the reference color information is a reference spectrum which is a spectrum of the reference color, and
wherein the calculation unit is configured to calculate the subject response based on the reference spectrum and the subject model, and to calculate the reference response based on the reference spectrum and the reference model.

### (Appendix 5)

The color evaluation system according to Appendix 4,
wherein the subject model includes a sensitivity function indicating sensitivity characteristics of each of L cones, M cones, and S cones of the subject,
wherein the reference model includes a sensitivity function indicating sensitivity characteristics of each of L cones, M cones, and S cones of the reference observer,
wherein the calculation unit is configured to:
   calculate the subject response indicating responses from each of L cones, M cones, and S cones of the subject based on the reference spectrum and the sensitivity function of the subject; and
   calculate the reference response indicating responses from each of L cones, M cones, and S cones of the reference observer based on the reference spectrum and the sensitivity function of the reference observer, and
wherein the comparison unit is configured to:
   calculate a difference between the subject response and the reference response for each of the L cones, the M cones, and the S cones; and
   calculate the response difference based on a combination of the difference for the L cones, the difference for the M cones, and the difference for the S cones.

### (Appendix 6)

The color evaluation system according to Appendix 4,
wherein the subject model includes a sensitivity function indicating a relationship between a color spectrum and a brain wave of the subject,
wherein the reference model includes a sensitivity function indicating a relationship between a color spectrum and a brain wave of the reference observer, and
wherein the calculation unit is configured to:
   calculate the subject response based on the brain wave of the subject based on the reference spectrum and the sensitivity function of the subject; and
   calculate the reference response based on the brain wave of the reference observer based on the reference spectrum and the sensitivity function of the reference observer.

### (Appendix 7)

The color evaluation system according to any one of Appendices 1 to 3,
wherein the acquisition unit is configured to acquire, as the reference color information, a steady state visual evoked potential of a subject who has visually recognized the reference color and a steady state visual evoked potential of a reference observer who has visually recognized the reference color, and
wherein the calculation unit is configured to calculate the subject response based on the steady state visual evoked potential of the subject and the subject model, and to calculate the reference response based on the steady state visual evoked potential of the reference observer and the reference model.

### (Appendix 8)

The color evaluation system according to Appendix 7,
wherein the subject model includes a sensitivity function indicating a ratio of a magnitude of the steady state visual evoked potential of the subject when the subject has visually recognized a change from the reference color to another predetermined color, to a magnitude of the steady state visual evoked potential of the subject when the subject has visually recognized a change from a predetermined first gray to a predetermined second gray,
wherein the reference model includes the sensitivity function indicating a ratio of a magnitude of the steady state visual evoked potential of the reference observer when the reference observer has visually recognized a change from the reference color to the other color, to a magnitude of the steady state visual evoked potential of the reference observer when the reference observer has visually recognized a change from the first gray to the second gray, and
wherein the calculation unit is configured to:
   calculate the ratio of the subject as the subject response based on the steady state visual evoked potential of the subject and the sensitivity function of the subject; and
   calculate the ratio of the reference observer as the reference response based on the steady state visual evoked potential of the reference observer and the sensitivity function of the reference observer.

### (Appendix 9)

The color evaluation system according to Appendix 7,
wherein the subject model indicates a relationship between a sample color and a steady state visual evoked potential of the subject who has visually recognized the sample color,
wherein the reference model indicates a relationship between the sample color and a steady state visual evoked potential of the reference observer who has visually recognized the sample color, and
wherein the calculation unit is configured to:
   calculate, using the subject model, the sample color corresponding to the steady state visual evoked potential of the subject acquired by the acquisition unit as the subject response; and
   calculate, using the reference model, the sample color corresponding to the steady state visual evoked potential of the reference observer acquired by the acquisition unit as the reference response.

### (Appendix 10)

The color evaluation system according to Appendix 9,
wherein the subject model includes a database indicating the relationship between the sample color and the steady state visual evoked potential of the subject, and
wherein the reference model includes a database indicating the relationship between the sample color and the steady state visual evoked potential of the reference observer.

### (Appendix 11)

The color evaluation system according to Appendix 9,
wherein the subject model includes a trained model that receives an input of the steady state visual evoked potential of the subject and outputs the sample color, and
wherein the reference model includes a trained model that receives an input of the steady state visual evoked potential of the reference observer and outputs the sample color.

### (Appendix 12)

The color evaluation system according to Appendix 4,
wherein the subject model includes a sensitivity function indicating a relationship between a color spectrum and an amount of change in pupil size of the subject,
wherein the reference model includes a sensitivity function indicating a relationship between a color spectrum and an amount of change in pupil size of the reference observer, and
wherein the calculation unit is configured to:
   calculate the subject response based on the amount of change in pupil size of the subject based on the reference spectrum and the sensitivity function of the subject; and
   calculate the reference response based on the amount of change in pupil size of the reference observer based on the reference spectrum and the sensitivity function of the reference observer.

### (Appendix 13)

The color evaluation system according to Appendix 4,
wherein the subject model includes a sensitivity function indicating a relationship between a color spectrum and an amount of change in cerebral blood flow of the subject,
wherein the reference model includes a sensitivity function indicating a relationship between a color spectrum and an amount of change in cerebral blood flow of the reference observer, and
wherein the calculation unit is configured to:
   calculate the subject response based on the amount of change in cerebral blood flow of the subject based on the reference spectrum and the sensitivity function of the subject; and
   calculate the reference response based on the amount of change in cerebral blood flow of the reference observer based on the reference spectrum and the sensitivity function of the reference observer.

### (Appendix 14)

The color evaluation system according to Appendix 4,
wherein the subject model includes a sensitivity function indicating a relationship between a color spectrum and an amount of change in amylase concentration of the subject,
wherein the reference model includes a sensitivity function indicating a relationship between a color spectrum and an amount of change in amylase concentration of the reference observer, and
wherein the calculation unit is configured to:
   calculate the subject response based on the amount of change in amylase concentration of the subject based on the reference spectrum and the sensitivity function of the subject; and
   calculate the reference response based on the amount of change in amylase concentration of the reference observer based on the reference spectrum and the sensitivity function of the reference observer.

### (Appendix 15)

The color evaluation system according to Appendix 4,
wherein the subject model includes a sensitivity function indicating a relationship between a color spectrum and an amount of change in gaze of the subject,
wherein the reference model includes a sensitivity function indicating a relationship between a color spectrum and an amount of change in gaze of the reference observer, and
wherein the calculation unit is configured to:
   calculate the subject response based on the amount of change in gaze of the subject based on the reference spectrum and the sensitivity function of the subject; and
   calculate the reference response based on the amount of change in gaze of the reference observer based on the reference spectrum and the sensitivity function of the reference observer.

### (Appendix 16)

The color evaluation system according to Appendix 4,
wherein the subject model includes a sensitivity function indicating a relationship between a color spectrum and an amount of change in skin potential of the subject,
wherein the reference model includes a sensitivity function indicating a relationship between a color spectrum and an amount of change in skin potential of the reference observer, and
wherein the calculation unit is configured to:
   calculate the subject response based on the amount of change in skin potential of the subject based on the reference spectrum and the sensitivity function of the subject; and
   calculate the reference response based on the amount of change in skin potential of the reference observer based on the reference spectrum and the sensitivity function of the reference observer.

### (Appendix 17)

A color evaluation method executed by a color evaluation system including at least one processor, the color evaluation method comprising:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model; and
calculating a difference between the subject response and the reference response as a response difference.

### (Appendix 18)

A method for producing a colorant, the method comprising:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model;
calculating a difference between the subject response and the reference response as a response difference;
generating, based on a reference spectrum which is a spectrum of the reference color and the response difference, an adjusted spectrum, which is a spectrum of a color having a response difference smaller than that of the reference spectrum; and
producing a colorant based on the adjusted spectrum.

### (Appendix 19)

A color evaluation program causing a computer to execute:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model; and
calculating a difference between the subject response and the reference response as a response difference.

According to Appendices 1, 17, and 19, the recognition of the reference color by the subject is calculated as the subject response, the recognition of the reference color by the reference observer is calculated as the reference response, and the difference between these two responses is calculated as the response difference. The response difference quantitatively indicates the discrepancy in recognition of the reference color between the subject and the reference observer. Therefore, by using the response difference, it becomes possible to quantitatively evaluate a color (reference color) from the perspective of color vision. In one example, by such a mechanism, people who feel barriers to empathizing with others or enjoying colors together due to being persons with color vision deficiency are enabled to enjoy colors together with others. In addition, it becomes possible to develop colors such that the appearance becomes closer between persons with color vision deficiency and persons with normal color vision. This makes it possible to obtain indicators for developing colorants such as dyes and paints, or products utilizing such colorants (for example, apparel products and writing instruments). Ultimately, it becomes possible to build a world in which both persons with color vision deficiency and persons with normal color vision can enjoy products while sharing the same color perception.

According to Appendix 2, since a color is automatically evaluated based on the response difference, a user can easily know how a color has been evaluated from the perspective of human color vision.

According to Appendix 3, an adjusted spectrum having a response difference smaller than that of the reference spectrum is generated. By using the adjusted spectrum, it becomes possible to provide colors in consideration of color vision diversity. For example, it becomes possible to generate and provide a color that is recognized in the same manner by both a person with normal color vision and a person with color vision deficiency.

According to Appendix 4, the recognition of the reference color by the subject is calculated as the subject response, the recognition of the reference color by the reference observer is calculated as the reference response, and the difference between these two responses is calculated as the response difference. The response difference quantitatively indicates the discrepancy in recognition of the reference color between the subject and the reference observer. Therefore, by using the response difference, it becomes possible to quantitatively evaluate a color (reference color) from the perspective of color vision.

According to Appendix 5, since the subject model and the reference model that simulate the actual human color vision system are used, the subject response and the reference response can be calculated more accurately. Therefore, a more accurate response difference can be obtained.

According to Appendix 6, since the subject model and the reference model that simulate actual human brain waves are used, the subject response and the reference response can be calculated more accurately. Therefore, a more accurate response difference can be obtained.

According to Appendix 7, the subject response and the reference response are calculated based on visual reactions in the human brain. By this approach, since the subject response and the reference response that more directly indicate the recognition of the reference color by a human are obtained, it can be expected that a response difference that more accurately represents the actual discrepancy in recognition is obtained.

According to Appendix 8, since sensitivity functions that take into account visual reactions in the human brain are used as the subject model and the reference model, the subject response and the reference response can be calculated more accurately. Therefore, a more accurate response difference can be obtained.

According to Appendix 9, since the relationship between a color (sample color) and a steady state visual evoked potential (SSVEP) is used as the subject model and the reference model in consideration of visual reactions in the human brain, the subject response and the reference response can be calculated more accurately. Therefore, a more accurate response difference can be obtained. In addition, since the subject response and the reference response are represented by colors (sample colors), these responses can be obtained in an easy-to-understand or intuitive manner.

According to Appendix 10, since the relationship between a color (sample color) and a steady state visual evoked potential (SSVEP) is prepared in the form of a database, the subject response and the reference response can be easily obtained by a simple procedure of referring to the database.

According to Appendix 11, since the relationship between a color (sample color) and a steady state visual evoked potential (SSVEP) is prepared by a trained model, it can be expected that a highly accurate subject response and reference response are obtained.

According to Appendix 12, since the subject model and the reference model that simulate actual pupil size of a human are used, the subject response and the reference response can be calculated more accurately. Therefore, a more accurate response difference can be obtained.

According to Appendix 13, since the subject model and the reference model that simulate actual cerebral blood flow of a human are used, the subject response and the reference response can be calculated more accurately. Therefore, a more accurate response difference can be obtained.

According to Appendix 14, since the subject model and the reference model that simulate actual amylase concentration of a human are used, the subject response and the reference response can be calculated more accurately. Therefore, a more accurate response difference can be obtained.

According to Appendix 15, since the subject model and the reference model that simulate actual changes in gaze of a human are used, the subject response and the reference response can be calculated more accurately. Therefore, a more accurate response difference can be obtained.

According to Appendix 16, since the subject model and the reference model that simulate actual changes in human skin potential are used, the subject response and the reference response can be calculated more accurately. Therefore, a more accurate response difference can be obtained.

According to Appendix 18, the recognition of the reference color by the subject is calculated as the subject response, the recognition of the reference color by the reference observer is calculated as the reference response, and the difference between these two responses is calculated as the response difference. Then, an adjusted spectrum having a response difference smaller than that of the reference spectrum is generated, and a colorant is produced based on the adjusted spectrum. By using the colorant expected to be recognized in the same manner by both the subject and the reference observer, it becomes possible to provide colors in consideration of color vision diversity.

### Reference Signs List

10: Color evaluation system; 11: Acquisition unit; 12: Calculation unit; 13: Comparison unit; 14: Evaluation unit; 15: Generation unit.

## Claims

1. A color evaluation system comprising:
an acquisition unit configured to acquire reference color information relating to a reference color, a subject model indicating recognition of colors by a subject, and a reference model indicating recognition of colors by a reference observer;
a calculation unit configured to calculate a subject response indicating recognition of the reference color by the subject based on the reference color information and the subject model, and to calculate a reference response indicating recognition of the reference color by the reference observer based on the reference color information and the reference model; and
a comparison unit configured to calculate a difference between the subject response and the reference response as a response difference.

2. The color evaluation system according to claim 1, further comprising an evaluation unit configured to evaluate the reference color based on the response difference.

3. The color evaluation system according to claim 1, further comprising a generation unit configured to generate, based on a reference spectrum which is a spectrum of the reference color and the response difference, an adjusted spectrum, which is a spectrum of a color having a response difference smaller than that of the reference spectrum.

4. The color evaluation system according to any one of claims 1 to 3,
wherein the reference color information is a reference spectrum which is a spectrum of the reference color, and
wherein the calculation unit is configured to calculate the subject response based on the reference spectrum and the subject model, and to calculate the reference response based on the reference spectrum and the reference model.

5. The color evaluation system according to claim 4,
wherein the subject model includes a sensitivity function indicating sensitivity characteristics of each of L cones, M cones, and S cones of the subject,
wherein the reference model includes a sensitivity function indicating sensitivity characteristics of each of L cones, M cones, and S cones of the reference observer,
wherein the calculation unit is configured to:
calculate the subject response indicating responses from each of L cones, M cones, and S cones of the subject based on the reference spectrum and the sensitivity function of the subject; and
calculate the reference response indicating responses from each of L cones, M cones, and S cones of the reference observer based on the reference spectrum and the sensitivity function of the reference observer, and
wherein the comparison unit is configured to:
calculate a difference between the subject response and the reference response for each of the L cones, the M cones, and the S cones; and
calculate the response difference based on a combination of the difference for the L cones, the difference for the M cones, and the difference for the S cones.

6. The color evaluation system according to claim 4,
wherein the subject model includes a sensitivity function indicating a relationship between a color spectrum and a brain wave of the subject,
wherein the reference model includes a sensitivity function indicating a relationship between a color spectrum and a brain wave of the reference observer, and
wherein the calculation unit is configured to:
calculate the subject response based on the brain wave of the subject based on the reference spectrum and the sensitivity function of the subject; and
calculate the reference response based on the brain wave of the reference observer based on the reference spectrum and the sensitivity function of the reference observer.

7. The color evaluation system according to any one of claims 1 to 3,
wherein the acquisition unit is configured to acquire, as the reference color information, a steady state visual evoked potential of a subject who has visually recognized the reference color and a steady state visual evoked potential of a reference observer who has visually recognized the reference color, and
wherein the calculation unit is configured to calculate the subject response based on the steady state visual evoked potential of the subject and the subject model, and to calculate the reference response based on the steady state visual evoked potential of the reference observer and the reference model.

8. The color evaluation system according to claim 7,
wherein the subject model includes a sensitivity function indicating a ratio of a magnitude of the steady state visual evoked potential of the subject when the subject has visually recognized a change from the reference color to another predetermined color, to a magnitude of the steady state visual evoked potential of the subject when the subject has visually recognized a change from a predetermined first gray to a predetermined second gray,
wherein the reference model includes the sensitivity function indicating a ratio of a magnitude of the steady state visual evoked potential of the reference observer when the reference observer has visually recognized a change from the reference color to the other color, to a magnitude of the steady state visual evoked potential of the reference observer when the reference observer has visually recognized a change from the first gray to the second gray, and
wherein the calculation unit is configured to:
calculate the ratio of the subject as the subject response based on the steady state visual evoked potential of the subject and the sensitivity function of the subject; and
calculate the ratio of the reference observer as the reference response based on the steady state visual evoked potential of the reference observer and the sensitivity function of the reference observer.

9. The color evaluation system according to claim 7,
wherein the subject model indicates a relationship between a sample color and a steady state visual evoked potential of the subject who has visually recognized the sample color,
wherein the reference model indicates a relationship between the sample color and a steady state visual evoked potential of the reference observer who has visually recognized the sample color, and
wherein the calculation unit is configured to:
calculate, using the subject model, the sample color corresponding to the steady state visual evoked potential of the subject acquired by the acquisition unit as the subject response; and
calculate, using the reference model, the sample color corresponding to the steady state visual evoked potential of the reference observer acquired by the acquisition unit as the reference response.

10. The color evaluation system according to claim 9,
wherein the subject model includes a database indicating the relationship between the sample color and the steady state visual evoked potential of the subject, and
wherein the reference model includes a database indicating the relationship between the sample color and the steady state visual evoked potential of the reference observer.

11. The color evaluation system according to claim 9,
wherein the subject model includes a trained model that receives an input of the steady state visual evoked potential of the subject and outputs the sample color, and
wherein the reference model includes a trained model that receives an input of the steady state visual evoked potential of the reference observer and outputs the sample color.

12. The color evaluation system according to claim 4,
wherein the subject model includes a sensitivity function indicating a relationship between a color spectrum and an amount of change in pupil size of the subject,
wherein the reference model includes a sensitivity function indicating a relationship between a color spectrum and an amount of change in pupil size of the reference observer, and
wherein the calculation unit is configured to:
calculate the subject response based on the amount of change in pupil size of the subject based on the reference spectrum and the sensitivity function of the subject; and
calculate the reference response based on the amount of change in pupil size of the reference observer based on the reference spectrum and the sensitivity function of the reference observer.

13. The color evaluation system according to claim 4,
wherein the subject model includes a sensitivity function indicating a relationship between a color spectrum and an amount of change in cerebral blood flow of the subject,
wherein the reference model includes a sensitivity function indicating a relationship between a color spectrum and an amount of change in cerebral blood flow of the reference observer, and
wherein the calculation unit is configured to:
calculate the subject response based on the amount of change in cerebral blood flow of the subject based on the reference spectrum and the sensitivity function of the subject; and
calculate the reference response based on the amount of change in cerebral blood flow of the reference observer based on the reference spectrum and the sensitivity function of the reference observer.

14. The color evaluation system according to claim 4,
wherein the subject model includes a sensitivity function indicating a relationship between a color spectrum and an amount of change in amylase concentration of the subject,
wherein the reference model includes a sensitivity function indicating a relationship between a color spectrum and an amount of change in amylase concentration of the reference observer, and
wherein the calculation unit is configured to:
calculate the subject response based on the amount of change in amylase concentration of the subject based on the reference spectrum and the sensitivity function of the subject; and
calculate the reference response based on the amount of change in amylase concentration of the reference observer based on the reference spectrum and the sensitivity function of the reference observer.

15. The color evaluation system according to claim 4,
wherein the subject model includes a sensitivity function indicating a relationship between a color spectrum and an amount of change in gaze of the subject,
wherein the reference model includes a sensitivity function indicating a relationship between a color spectrum and an amount of change in gaze of the reference observer, and
wherein the calculation unit is configured to:
calculate the subject response based on the amount of change in gaze of the subject based on the reference spectrum and the sensitivity function of the subject; and
calculate the reference response based on the amount of change in gaze of the reference observer based on the reference spectrum and the sensitivity function of the reference observer.

16. The color evaluation system according to claim 4,
wherein the subject model includes a sensitivity function indicating a relationship between a color spectrum and an amount of change in skin potential of the subject,
wherein the reference model includes a sensitivity function indicating a relationship between a color spectrum and an amount of change in skin potential of the reference observer, and
wherein the calculation unit is configured to:
calculate the subject response based on the amount of change in skin potential of the subject based on the reference spectrum and the sensitivity function of the subject; and
calculate the reference response based on the amount of change in skin potential of the reference observer based on the reference spectrum and the sensitivity function of the reference observer.

17. A color evaluation method executed by a color evaluation system including at least one processor, the color evaluation method comprising:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model; and
calculating a difference between the subject response and the reference response as a response difference.

18. A method for producing a colorant, the method comprising:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model;
calculating a difference between the subject response and the reference response as a response difference;
generating, based on a reference spectrum which is a spectrum of the reference color and the response difference, an adjusted spectrum, which is a spectrum of a color having a response difference smaller than that of the reference spectrum; and
producing a colorant based on the adjusted spectrum.

19. A color evaluation program causing a computer to execute:
acquiring reference color information relating to a reference color, a subject model indicating recognition of colors by a subject, and a reference model indicating recognition of colors by a reference observer;
calculating a subject response indicating recognition of the reference color by the subject based on the reference color information and the subject model, and calculating a reference response indicating recognition of the reference color by the reference observer based on the reference color information and the reference model; and
calculating a difference between the subject response and the reference response as a response difference.
